Europäisches Patentamt

European Patent Office

Office européen des brevets

(11) Publication number: **0 099 650**
**B1**

(12) **EUROPEAN PATENT SPECIFICATION**

(45) Date of publication of patent specification: **26.02.86**

(51) Int. Cl.[4]: **C 07 C 1/20,** C 10 G 3/00

(21) Application number: **83303480.4**

(22) Date of filing: **16.06.83**

(54) **Two-stage, zeolite catalyzed process for the conversion of alcohols to hydrocarbons.**

(30) Priority: **20.07.82 US 400161**

(43) Date of publication of application:
**01.02.84 Bulletin 84/05**

(45) Publication of the grant of the patent:
**26.02.86 Bulletin 86/09**

(84) Designated Contracting States:
**BE DE FR GB IT NL**

(56) References cited:
**US-A-4 046 825**
**US-A-4 238 631**

(73) Proprietor: **MOBIL OIL CORPORATION**
**150 East 42nd Street**
**New York New York 10017 (US)**

(72) Inventor: **Gould, Ronald Michael**
**223 Champion Way**
**Sewell New Jersey 08080 (US)**
Inventor: **Yuk-Yim Kam, Anthony**
**18 S. Birchwood Park Drive**
**Cherry Hill New Jersey 08003 (US)**

(74) Representative: **West, Alan Harry**
**Mobil Court 3 Clements Inn**
**London WC2A 2EB (GB)**

Courier Press, Leamington Spa, England.

## Description

This invention relates to a two-stage, zeolite catalyzed process for the conversion of alcohols to hydrocarbons.

The growing demand for gasoline and other light petroleum products, including light gases, and the growing shortage of crude oil, has resulted in a continuing interest in tapping alternate raw material sources from which to obtain the desired light hydrocarbon products.

In recent years it has been disclosed that methanol, which may be obtained from coal, natural gas or biomass, can be converted to more complex hydrocarbons, including gasoline boiling range hydrocarbons, by utilizing a particular group of zeolite catalysts, exemplified by ZSM-5 zeolite. In one commercial aspect of this process, natural gas is converted to gasoline employing Mobil Oil Corporation's methanol to gasoline (MTG process after the natural gas is converted to synthesis gas and then methanol. The MTG process is disclosed in, for example, U.S. Patent Nos. 3,894,103; 3,894,104; 3,894,107; 4,035,430 and 4,058,576. U.S. Patent No. 3,894,102 discloses the conversion of synthesis gas to gasoline.

The conversion of lower monohydric alcohols to gasoline by the catalytic action of ZSM-5 type zeolites is highly exothermic. A large body of patent art is concerned with various techniques for controlling the heat released in this process. For example, U.S. Patent No. 3,931,349 discloses the addition of a light hydrocarbon diluent to the reactants to act as a heat sink for the exothermic heat released when the reaction mixture is contacted with the conversion catalyst.

U.S. Patent No. 4,035,430 describes arranging the catalyst in a series of beds of increasing size with interstage quenching with methanol, dimethyl ether and/or light hydrocarbons for controlling exothermic heat. A tubular reactor is disclosed in U.S. Patent No. 4,058,576 as a means of removing exothermic heat during the catalytic conversion of a lower alcohol to gasoline.

To control the exothermic heat released in this conversion of alcohols, suggestions have also been made to employ fluidized catalyst techniques which were developed heretofore, principally in the petroleum industry. Fluidization methods were especially usefully employed in the cracking of gas oils where the powdered catalyst was used to transfer heat developed during the regeneration of the catalyst in a fluidized bed to effect the endothermic cracking of the gas oil in another fluidized bed of catalyst.

It has also been proposed to use the fluidized catalyst technique in the highly exothermic reactions of Fischer-Tropsch synthesis and the known Oxo process and other such exothermic processes primarily for the disposal of generated heat. In the fluidized catalyst operations developed, disposal of the reaction heat has been accomplished by many different techniques including transfer of catalyst through cooling sections and/or indirect cooling with liquids or a fluid catalyst to absorb reaction heat transferred directly or indirectly by the finely divided fluidized catalyst particles.

Fluidized catalyst techniques have also been employed to control the exothermic heat released in the conversion of alcohols to gasoline. Thus, U.S. Patent No. 4,071,573 discloses the use of dilute catalyst phase riser reactors and dense catalyst phase reactors together with external indirect heat exchange means for disposing of exothermic reaction heat to prolong the useful life of the ZSM-5 type catalyst. Heat control and reactant plug flow conditions are achieved in a dense fluid bed reactor in U.S. Patent No. 4,197,418 by providing vertical baffles in the reactor and external catalyst coolers. U.S. Patent No. 4,238,631 employs a riser reactor and a dense fluid catalyst bed in series, together with satellite stripping cooling zones, in a fashion which not only controls the heat release but also limits the production of durene. Both vertical baffles and vertical heat exchanger tubes are provided in the dense fluid bed catalytic reactor employed to convert lower alcohols to gasoline boiling hydrocarbons in U.S. Patent No. 4,251,484. The configuration of the baffles and tubes provides a flow path within a specified hydraulic diameter range to restrict gas bubble growth while the internal heat exchanger permits the generation of steam to control the exothermic heat release.

All of the fluid bed alcohol conversion processes are characterized by virtually complete (99.5%) conversion of a methanol feed to gasoline range hydrocarbons. Exothermic heat of reaction is removed by internal steam generation or by circulation of catalyst to an external vessel where heat removal is effected. Further a portion of the conversion catalyst is continuously withdrawn from reactor for partial regeneration. To achieve the 99.5% conversion specification in the prior art fluid bed reactors, relatively low weight hourly space velocities (about 1.0) and the use of extensive reactor baffling are often required. Although gasoline yields of 88—90 weight percent are reported, downstream alkylation capacity of approximately 0.3 l/l of final product is required.

It is an object of this invention to improve the prior art processes which employ fluid bed technology for the conversion of lower alcohols to gasoline boiling range hydrocarbons.

In accordance with the present invention, there is provided a process for converting an alcohol-containing reactant to a hydrocarbon product having a higher boiling range than the reactant and comprising gasoline boiling range hydrocarbons which comprises:

(a) contacting the reactant with a first quantity of a ZSM-5 type crystalline zeolite catalyst in a fluid bed reaction zone under exothermic reaction conditions to effect an alcohol conversion of 80 to 95% and release of 80—95% of the total exothermic heat released during substantially complete conversion of the alcohol.

(b) separating the product from the fluid bed reaction zone from the first quantity of ZSM-5 type catalyst,

(c) passing a first portion of the first quantity of ZSM-5 type catalyst through a catalyst cooling zone under cooling conditions and thence back to the fluid bed reaction zone,

(d) passing a second portion of the first quantity of ZSM-5 type catalyst through a catalyst regeneration zone and thence back to the fluid bed reaction zone, characterized by including the further steps of:

(e) contacting the product from the fluid bed reaction zone with a second quantity of a ZSM-5 type crystalline zeolite catalyst in a fixed bed reaction zone under exothermic reaction conditions effective to substantially complete alcohol conversion and release of 5—20% of the total exothermic heat released during substantially complete conversion of said lower alcohol conversion and,

(f) recovering a reactant product comprising $C_1$—$C_4$ light hydrocarbon gases and $C_5+$ gasoline boiling range hydrocarbons from the fixed bed reaction zone.

The feed to the process of this invention may include a single lower alcohol alone or a mixture of lower alcohols. The feed may also include admixtures of lower alcohol or alcohols with related oxygenates and/or water. Examples of related oxygenates are the ethers of the useful alcohols, i.e., dimethyl ether is a related oxygenate of methanol. Water may comprise 0 to about 50 weight percent of the feed. Preferably the lower alcohols include $C_1$—$C_4$ monohydric alcohols and $C_2$—$C_4$ dihydric alcohols. The monohydric alcohols are the preferred feedstocks in this process.

The $C_1$—$C_4$ monohydric alcohols that may be charged to the process of this invention, include methanol, ethanol, propanol, isopropanol, and the butanols, with methanol being preferred. The $C_2$—$C_4$ dihydric alcohols that may be used include ethylene glycol, the propanediols and the butanediols. In general, any mixture which comprises methanol, ethanol, propanol, isopropanol, a butanol, ethylene glycol, a propanediol, a butanediol, or mixtures thereof and which is convertible with high exothermicity, is a suitable feed for the present invention. Conversions which produce more than 232600 J/kg (100 BTU/lb) of total hydrocarbon product, and preferably more than 465200 J/kg (200 BTU/lb) of hydrocarbon product, at the conversion temperature, are considered highly exothermic. $C_4+$ mono and dihydric alcohols may serve as feed in the process of this invention. However, these alcohols usually are more valuable for other uses than to serve as gasoline precursors and thus are not preferred feedstocks.

A preferred charge is methanol together with 0 to 50 weight percent water. A particularly preferred charge is a methanol-water mixture where the water content does not exceed 20 weight percent, based on the admixture. Mixtures of methanol and dimethyl ether are also preferred charges.

In the following description, the process will be described with methanol serving as the feedstream. This is for convenience and for illustrative purposes only since it will be appreciated that the feed can comprise any of the other alcohols described herein either alone or admixed with other alcohols, or oxygenates thereof as well as water.

The conversion is conducted in the presence of a crystalline zeolite catalyst of the ZSM-5 type. The ZSM-5 type zeolite catalysts used herein are crystalline zeolites having a silica/alumina ratio greater than 12 and a Constraint Index (C.I.) between 1 and 12. These zeolites and their use as conversion catalysts for lower aliphatic alcohols are described in, for example, U.S. Patent Nos. 3,894,106, 4,025,571, 4,058,576 and 4,148,835.

The ZSM-5 type zeolites described above are exemplified by ZSM-5, ZSM-11, ZSM-12, ZSM-23, ZSM-35 and ZSM-38, with ZSM-5 being particularly preferred.

ZSM-5 is more particularly described in U.S. Patent No. 3,702,886. ZSM-11 is more particularly described in U.S. Patent No. 3,709,979. ZSM-12 is more particularly described in U.S. Patent No. 3,832,449. ZSM-23 is more particularly described in U.S. Patent No. 4,076,842. ZSM-35 is more particularly described in U.S. Patent No. 4,016,245. ZSM-38 is more particularly described in U.S. Patent No. 4,046,859.

Particularly preferred catalysts within the above descriptions are ZSM-5 type zeolite catalysts having a large crystal size, i.e. a crystal size of at least 1 micron, as described in U.S. Patent Nos. 4,025,571 and 4,148,835.

Although the fluid catalyst processes of the prior art achieve up to 99.5 weight percent conversion of the alcohol in the feedstream to higher molecular weight hydrocarbons, the present process is intended to effect essentially 100 percent complete conversion of the methanol. This difference in yield may appear slight but those skilled in the art will appreciate that the substantial elimination of alcohol contamination in the aqueous product results in the consequent elimination of waste water treatment of this stream.

The present process employs two reactors in series. In the first reactor, a fluid bed operates at moderately high space velocities while achieving partial methanol conversion. Complete conversion of the unreacted methanol is readily accomplished in the second, fixed bed reactor. The fluid bed reactor is operated under substantially reactant plug flow conditions and exothermic heat of reaction is removed by internal steam generation, internal heat exchange or by circulation of catalyst to an external vessel where heat removal is effected. Further, a portion of the zeolite catalyst is continuously withdrawn from the reactor for partial regeneration. In the fixed bed reactor, removal of the heat of reaction is not generally required. However, heat removal may be accomplished by means of a light hydrocarbon gas diluent or by means of cooling coils in the bed.

The main fluid bed reactor may be operated in the turbulent dense bed mode or as a dilute phase riser reactor. In a further embodiment, a combination of dilute phase and dense bed conversion is employed. The initial reaction, conducted in the fluid bed reactor, is operated at 80—95 weight percent methanol conversion and generates 80—95% of the total exothermic heat released during complete conversion of the methanol. The higher conversion level is representative of a dense fluid bed reactor operating at an elevated weight hourly space velocity (WHSV) of 1.5 to 2.5. The lower conversion level is typical of riser reactor performance with space velocities in the range of 3.5 to 7.0.

The type of fluid bed reactor employed, viz, dense bed, dilute phase riser or combination thereof does not significantly affect the process. Rather it is the conversion obtained in the first reactor which is significant. All other things being equal, the same conversion obtained in the first reaction zone will provide substantially the same product slate for final conversion in the fixed bed reactor regardless of the type or types of fluidization employed in the first reaction zone.

In the accompanying drawings,

Figure 1 is a schematic flowplan of a first embodiment of the invention employing a dilute catalyst phase riser reactor,

Figure 2 is a schematic flowplan of a second embodiment of the invention employing a dense fluid catalyst bed reactor, and

Figure 3 is a schematic flowplan of a third embodiment of the invention employing a dilute catalyst phase riser reactor followed by a dense fluid catalyst bed reactor.

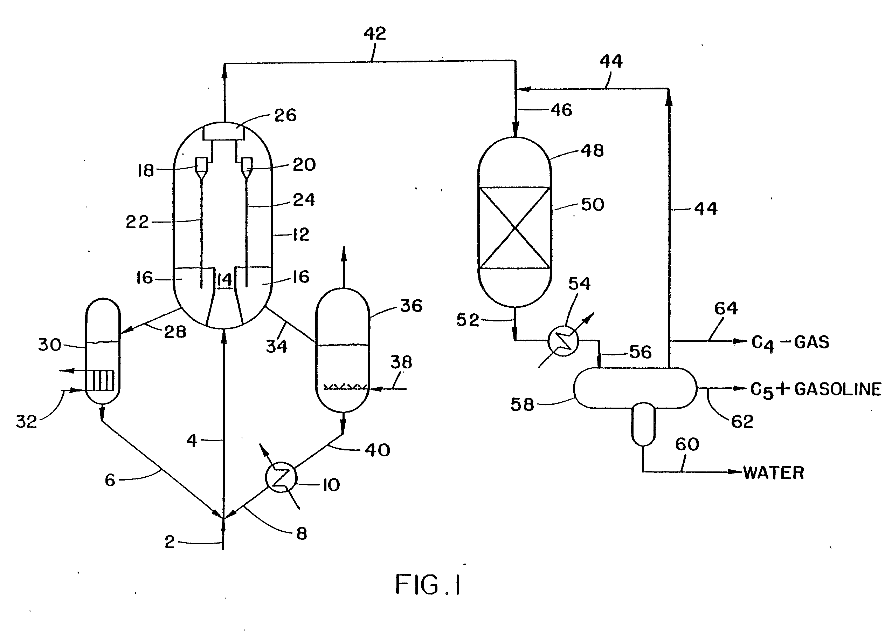

Referring now to Figure 1, in the first embodiment of this invention a methanol feed containing 0—20 weight percent water, is introduced preferably in the vaporous state, through line 2 into the lower end of riser reactor 4 where it is combined with cooled recycle ZSM-5 zeolite catalyst from line 6 and freshly regenerated catalyst from line 8. The mixture of methanol feed and catalyst forms a suspension at a temperature in the range 204—427°C (400—800°F). The temperature may be regulated by employing cooler 10 to adjust the temperature of the regenerated catalyst and/or by regulating the heat removed by heat exchange means 32. The suspension of methanol reactant and zeolite catalyst then passes upwardly through riser 4 under conversion conditions effective to achieve a conversion in the range 80 to 90 weight percent. Useful operating conditions include a WHSV of 3.5 to 7.0, a residence time of 1 to 10 seconds, a catalyst density of 16—240 kg/m³ (1 to 15 pounds per cubic foot) and a catalyst to methanol weight ratio greater than 20, preferably 30 to 60 and a maximum temperature of 427°C (800°F). The riser may be provided with cross-sectional dispersing means, such as that disclosed in U.S. Patent No.

4,071,573, to disperse any gas bubbles formed in the suspension. This reduces backmixing and assures substantial plug flow conditions in the riser.

The suspension discharges from riser 4 into disengaging vessel 12 where it passes through conical section 14 into the upper section of vessel 12 where the enlarged diameter of the vessel causes a substantial reduction in velocity resulting in a separation of the suspension into a vapor phase and a catalyst phase. A body of catalyst 16 collects in the lower end of vessel 12. A vapor phase passes through catalyst separation means 18 and 20 which remove entrained catalyst from the vapors and conduct it through diplegs 22 and 24 to the body of catalyst 16.

The catalyst separation means must be adequate to prevent carryover of the finely divided fluid catalyst particles to the fixed bed thereby eliminating excessive pressure drop build-up across the fixed bed of larger catalyst particles which would otherwise result. Those skilled in the art will appreciate that such devices as filters, electrostatic precipitators and the like, alone or in combination with cyclone separators, may be employed as the catalyst separation means.

The vapors exit the catalyst separation means and pass into plenum chamber 26. A portion of the catalyst is removed from vessel 12 through line 28 and passes to cooler vessel 30 where the exothermic heat of reaction released in the riser reactor is removed. The hot catalyst contacts heat exchanger 32 causing water flowing therein to turn to high pressure steam which exits from the exchanger for use elsewhere. The cooled catalyst passes from the lower end of cooler vessel 30 into line 6 where it is recycled to riser 4.

A portion of the catalyst is also withdrawn through line 34 and introduced into regenerator 36. Generally about 5 to 10% of the catalyst inventory is withdrawn per hour. Oxygen containing gas, introduced through line 38 into the lower end of the regenerator through a distribution grid, passes upward through the body of catalyst causing the removal of at least part of the coke deposited thereon. As disclosed in U.S. Patent No. 4,238,631 a reduction of the weight percent coke on catalyst, preferably to a level of 2 to 20 weight percent, will provide a catalyst inventory of the requisite activity to achieve the desired conversion. Since processes for the oxidative regeneration of zeolite catalysts are well known details of the regeneration will not be described. Any of these procedures which are effective may be employed here. The regenerated catalyst passes from regenerator 36 through line 40 from which it is introduced into riser 4 after being cooled to the required temperature in cooler 10.

Returning to plenum chamber 26 in the upper end of vessel 12, the vapor phase passing therethrough contains the partially converted reactants and their products. This vapor phase which comprises methanol, dimethyl ether, water, and hydrocarbons passes from plenum chamber 26 through line 42 where it is admixed, if required, with a light hydrocarbon gas stream flowing through line 44. This light gas comprises a portion of the products recovered downstream in the subject process. The gaseous admixture passes through line 46 to reactor 48 containing a fixed bed 50 of ZSM-5 zeolite catalyst. The function of the light hydrocarbons gas stream, where present, is to act as a heat sink for the exothermic heat released in the fixed bed of catalyst. Alternately, an internal heat exchanger can be located within the fixed bed of catalyst to remove the exothermic heat of reactor. The gaseous phase admixture passes through reactor 48 under conversion conditions effective to complete the desired conversion of substantially all of the methanol in the feedstream, i.e. 5 to 20% of the overall conversion takes place in fixed bed reactor 48.

By effecting most of the conversion in the first stage under fluidized catalyst conditions, the process duty requirements of the fixed bed are significantly less than if all of the conversion were conducted in the fixed bed. Therefore, a substantially lower operating pressure can be employed and the reactor pressure drop is greatly reduced vis-a-vis an all fixed bed conversion process. At 95% conversion in the fluid bed, the fixed bed adiabatic temperature rise is estimated at about 30.5°C (55°F) in the absence of light gas recycle. Fixed bed inlet pressure is 239 to 308 kPa (20 to 30 psig); pressure drop across the fixed bed will be less than 122 kPa (3 psig) at WHSV of 1.6. At an 80% fluid bed conversion level, the adiabatic temperature rise across the fixed bed is estimated at about 119°C (215°F). This temperature rise can be limited to 55.6°C (100°F) by using a 2:1 light gas recycle ratio at an inlet pressure to the fixed bed of 446 to 515 kPa (50 to 60 psig). This is significantly lower than the 9:1 recycle ratio and 2170 kPa (300 psig) inlet pressure often required in a fixed bed only process.

In the present two-stage conversion process, 80—95% of the exothermic heat of reaction is released in the fluid bed where it is easily removed because of the inherent heat transfer ability of the fluid bed. Since only 5—20% of the total reaction heat is released in the second stage fixed bed, the light gas recycle often employed to control the heat release in the fixed bed processes of the prior art can be greatly reduced or even eliminated.

The products from the reaction pass from reactor 48 through line 52 to cooler 54 where water vapors and the normally liquid hydrocarbons are condensed. All of the reaction products then pass through line 56 to separator vessel 58 where the products are separated into three phases, an aqueous phase which is drawn off through line 60, a $C_5+$ hydrocarbon phase, i.e., the desired gasoline boiling range hydrocarbons, which is removed through line 62 and a $C_4-$ light hydrocarbon gas stream which is removed through line 64. The $C_5+$ stream is conventionally transferred to gasoline blending facilities where it is processed as required. The $C_4-$ stream may also be subject to further processing, i.e., alkylation to provide further components for gasoline blending.

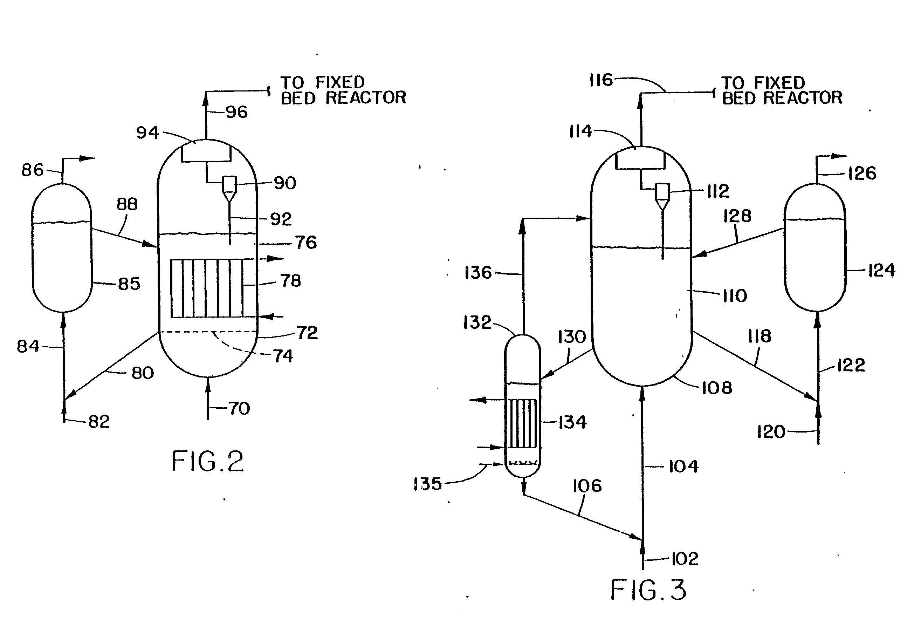

Several other embodiments of this invention are directed to alternatives in the first or fluid catalyst stage of the process. In the first embodiment, shown in Figure 1 and described above, the fluid catalyst is provided as a dilute phase in a riser reactor. In the second embodiment, as depicted in Figure 2, the initial conversion is effected in a dense bed fluid catalyst reactor. In the third embodiment, as depicted in Figure 3, a combination of dilute phase riser reactor conversion and dense bed fluid catalyst conversion is employed. In view of the similarity of the procedures employed in all three embodiments, the following description is directed only to the differences in the several alternatives and hence portions of the above description should be employed where applicable.

Referring to Figure 2, the process is conducted in essentially the same manner as in the first embodiment described above except that the catalyst in the first stage is provided as a dense fluid bed. Thus, the methanol feed, which again may contain 0—20 weight percent water and is most conveniently in a vapor state, is introduced through line 70 directly into a dense fluid bed reactor 72, such as that described in U.S. Patent No. 4,251,484. This fluid bed reactor is particularly adapted to methanol conversion and is equipped with heat exchanger means and baffle tube means to permit efficient conversion of the methanol feed. The feed stream passes through distributor grid 74, positioned across the lower portion of reactor 72 and enters the bottom of the dense fluid bed 76 of ZSM-5 catalyst. The catalyst density in the bed is between 32 and 641 kg/m³ (2 and 40 pounds per cubic foot). As the vaporous feed passes up through the fluid catalyst bed, the desired conversion releases exothermic heat of reaction which is removed by the generation of steam from water flowing through heat exchanger 78 located within the body of fluid catalyst. As coke is formed on the catalyst a portion of the catalyst in the bed is removed from reactor 72 through line 80 for regeneration. The procedure is similar to that described above. The coked catalyst is contacted by an oxygen-containing gas from line 82 and the resultant suspension passes up through line 84 into regeneration vessel 85 containing a bed of catalyst undergoing regeneration. A $CO_2$ rich gaseous stream containing the products of regeneration is removed through line 86. The partially regenerated catalyst is returned to reactor 72 through line 88.

The reaction mixture passing up through fluid catalyst bed 76 is converted to the desired degree and passes into the vapor space in the upper end of reactor vessel 72. The vaporous mixture passes through catalyst separation means 90 which removes entrained catalyst and returns it to the fluid bed of catalyst through diplet 92. The partially converted reaction mixture exits the catalyst separation means and enters plenum chamber 94 from which it exits reactor 72 through line 96 for passage to a fixed bed reactor. The methanol conversion is completed in the fixed bed and the products are recovered as described above in connection with the first embodiment.

The third embodiment combines the fluid catalyst concepts of the other two embodiments. Referring to Figure 3, the vaporous methanol feed is introduced through line 102 into the end of riser reactor 104 where it is combined with stripped and cooled ZSM-5 catalyst recycled through line 106. The mixture of catalyst and feed forms a suspension at a temperature of 204—427°C (400—800°F) and passes upward through riser 104 under appropriate conversion conditions. After the reaction mixture of feed and catalyst passes through riser 104 it discharges into reactor vessel 108 which contains dense fluid bed 110 of ZSM-5 catalyst. As the vaporous reaction mixture passes upward through the dense fluid catalyst, further conversion of the methanol takes place. The reaction conditions employed in the riser and the fluid bed are those described above and are selected so that the combined conversion is in the range of about 80—95 weight percent, leaving the rest of the required conversion to be achieved in the fixed bed reactor. As previously described, the vaporous reaction mixture passes into the vapor space at the upper end of reactor 108 where it enters catalyst separation means 112. Here entrained catalyst is separated and returned to fluid catalyst bed 110. The partially converted reaction mixture passes from the catalyst separation means into plenum chamber 114 and then into line 116 for transfer to the fixed bed reactor and the final conversion as described above.

As with the other embodiments, a portion of catalyst must be partially regenerated to maintain the coke on catalyst at the level which provides the required activity. Further, the exothermic heat of reaction must be removed. The catalyst can be utilized as a means to effect this. The catalyst is partially regenerated as described in the second embodiment by removing the catalyst from reactor 108 through line 118 and passing it, together with an oxygen-containing gas supplied through line 120, through line 122 into regenerator vessel 124 for removal of a portion of the coke. Flue gas is removed through line 126 and the partially regenerated catalyst is returned to the fluid bed reactor 108 through line 128. The heat of reaction can be removed by passing a portion of the catalyst from reactor 106 through line 130 to cooler 132. Here the catalyst is cooled by passing it in indirect heat exchange with water to generate steam in heat exchanger 134. In addition to being cooled, the catalyst may also be stripped of hydrocarbons in this vessel since it will be recycled for use in forming a dilute phase suspension with the fresh feed. Steam entering cooler 132 through line 135 passes upward through the catalyst in cooler 132 stripping occluded hydrocarbons from the catalyst. The steam and stripped materials pass from cooler 132 through line 136 into the vapor space of reactor 108. The partially regenerated and cooled catalyst passes from the lower end of cooler 132 through line 106 for recycle to riser reactor 104 where it forms a dilute phase suspension with fresh methanol feed.

The following example illustrates the practice of this invention.

Example I

A series of runs were made with a dense fluid bed reactor.

In run No. 1, a feed stream comprising 96 wt.% methanol and 4 wt.% water was reacted over ZSM-5 zeolite catalyst in a dense bed reactor having a catalyst density of 304 kg/m³ (19 pounds per cubic foot). The feed was passed upward through the reactor at a temperature of 413°C (775°F) and a WHSV which provided a residence time of about 12 seconds. The reaction mixture recovered from the top of the reactor was analyzed. A conversion of 80.8 weight percent was obtained. The product slate for this dense bed reactor conversion is shown in Table I below.

In a second run, the methanol/water, feed was passed into a dense fluid bed of ZSM-5 catalyst at a temperature of 413°C (775°F) and a WHSV of 1.9. The catalyst density in the bed was 368 kg/m³ (23 pounds per cubic foot). A conversion of 94.4 weight percent was obtained. The product slate for products from this dense bed is shown in Table I below.

A computer model of a ZSM-5 fixed bed reactor was employed to simulate the product slate for a total conversion of 100% employing the effluents of the first two runs as the feed to a fixed bed reactor. These simulated data are also presented in Table I. It should be noted that although the required fixed bed conversion was much higher when the conversion in the dense fluid bed of the first stage was 80.8%, the CO, $CO_2$, hydrocarbon and water yields are substantially the same as when the conversion in the dense fluid bed of first stage was 94.4% although the distribution among the hydrocarbons was different.

To compare the process of the subject invention with an all fluid bed process of the prior art, a third run was made which was similar to that of run No. 2 except that a high conversion of 99.5 weight percent was obtained. The product slate for this run is also presented in Table I. Comparing these data to those of the present invention shows that higher conversion and hydrocarbon yields are obtained by the process of the present invention.

TABLE I
Product yields
Integrated systems

| | Run No. 1 | | Run No. 2 | | Prior art |
| | Dilute fluid catalyst | Fixed bed | Dense phase fluid catalyst | Fixed bed | Run No. 3 Fluid bed |
| --- | --- | --- | --- | --- | --- |
| Conversion, Wt.% | 80.8 | 100 | 94.4 | 100 | 99.5 |
| **Compound** | | | | | |
| Methane | 0.79 | 0.55 | 0.48 | 0.41 | 0.32 |
| Ethane | 0.17 | 0.42 | 0.10 | 0.16 | 0.12 |
| Ethene | 3.29 | 0.04 | 1.96 | 0.01 | 1.32 |
| Propane | 1.12 | 3.33 | 0.68 | 2.13 | 1.79 |
| Propene | 9.14 | 0.29 | 5.57 | 0.08 | 3.25 |
| n-Butane | 1.00 | 1.56 | 0.63 | 1.08 | 0.80 |
| i-Butane | 3.04 | 3.70 | 1.94 | 3.65 | 4.31 |
| Butenes | 8.03 | 0.97 | 5.24 | 0.39 | 3.44 |
| $C_5+$ | 12.07 | 31.28 | 20.59 | 34.45 | 25.64 |

TABLE I (cont.)

| | Dilute fluid catalyst | Fixed bed | Dense phase fluid catalyst | Fixed bed | Fluid bed |
| --- | --- | --- | --- | --- | --- |
| **Compound** | | | | | |
| $H_2$ | .02 | — | — | — | .02 |
| CO | .06 | 0.02 | .05 | .01 | .06 |
| $CO_2$ | — | 0.03 | — | .02 | .02 |
| Hydrocarbons | 38.65 | 42.14 | 37.19 | 42.36 | 40.99 |
| Water | 34.78 | 57.81 | 53.78 | 57.68 | 57.85 |
| Methanol | 19.15 | — | 5.61 | — | .50 |
| Dimethyl Ether | 7.34 | — | 3.37 | — | .56 |
| | 100.0 | 100.0 | 100.0 | 100.0 | 100.0 |

The primary advantages of the combined fluid bed reactor and fixed bed reactor system of the present invention over the all fluid bed reactor system and the all fixed bed systems of the prior art are:

1. Reduction in the fluid bed operating severity, allowing increases in reactor space velocity (higher throughput, lower catalyst inventory) and ultimate catalyst life (less active catalyst). Operation in the dilute phase, riser reactor mode significantly enhances catalyst utilization while eliminating gas backmixing which contributes to durene formation.

2. The bulk of exothermic heat of reaction (80—95%) is released in the fluid bed where it is easily removed because of the superior heat transfer characteristics of the fluid bed.

3. Since only 5—20% of the total reaction heat is released in the second stage fixed bed, light gas recycle can be greatly reduced or eliminated.

4. In the second stage fixed bed, substantially lower operating pressures can be employed. Reactor pressure drop is greatly reduced.

5. Conversion of methanol in the two-stage reactor system is essentially 100% complete. Aqueous product contamination by methanol and

7

the effect on waste water treatment facilities are eliminated.

6. Substantial quantities of butanes are alkylated in the second stage fixed bed, thus reducing the size requirements of the downstream alkylation plant.

## Claims

1. A process for converting an alcohol-containing reactant to a hydrocarbon product having a higher boiling range than the reactant and comprising gasoline boiling range hydrocarbons which comprises:

(a) contacting the reactant with a first quantity of a ZSM-5 type crystalline zeolite catalyst in a fluid bed reaction zone under exothermic reaction conditions to effect an alcohol conversion of 80 to 95% and release of 80—95% of the total exothermic heat released during substantially complete conversion of the alcohol,

(b) separating the product from the fluid bed reaction zone from the first quantity of ZSM-5 type catalyst,

(c) passing a first portion of the first quantity of ZSM-5 type catalyst through a catalyst cooling zone under cooling conditions and thence back to the fluid bed reaction zone,

(d) passing a second portion of the first quantity of ZSM-5 type catalyst through a catalyst regeneration zone and thence back to the fluid bed reaction zone, characterized by including the further steps of:

(e) contacting the product from the fluid bed reaction zone with a second quantity of a ZSM-5 type crystalline zeolite catalyst in a fixed bed reaction zone under exothermic reaction conditions effective to substantially complete alcohol conversion and release of 5—20% of the total exothermic heat released during substantially complete conversion of said lower alcohol conversion and,

(f) recovering a reactant product comprising $C_1$—$C_4$ light hydrocarbon gases and $C_5$+ gasoline boiling range hydrocarbons from the fixed bed reaction zone.

2. A process according to claim 1 wherein the fluid bed reaction zone of step (a) is a dilute catalyst phase riser reaction zone.

3. A process according to claim 2 wherein the catalyst density in the dilute catalyst phase riser reaction zone is between 16 and 240 kg/m³ (1 and 15 pounds per cubic foot).

4. A process according to claim 1 wherein the fluid bed reaction zone of step (a) is a dense fluid catalyst bed reaction zone.

5. A process according to claim 3 wherein the catalyst density in the dense fluid catalyst bed reaction zone is between 32 and 641 kg/m³ (2 and 40 pounds per cubic foot).

6. A process according to claim 1 wherein the fluid bed reaction zone of step (a) is a dilute catalyst phase riser reaction zone in series with a dense fluid catalyst bed reaction zone.

7. A process according to any preceding claim wherein the reactant contains a $C_1$—$C_4$ monohydric alcohol, a $C_2$—$C_4$ dihydric alcohol, or mixture thereof with a related oxygenate.

8. A process according to claim 7 wherein the monohydric alcohol is methanol.

9. A process according to any preceding claim wherein the reactant contains 0—50 weight percent water.

10. A process according to any preceding claim wherein a portion of the $C_1$—$C_4$ hydrocarbon gases of step (f) are introduced into the fixed bed reaction zone at a rate effective to limit the temperature rise across the fixed bed reaction zone to no more than 56°C (100°F).

## Patentansprüche

1. Verfahren zur Umwandlung eines Alkohol enthaltenden Reaktants zu einem Kohlenwasserstoffprodukt mit einem höheren Siedebereich als das Reaktant, das Kohlenwasserstoffe des Benzin-Siedebereichs umfaßt, wobei das Verfahren umfaßt:

(a) Kontaktieren des Reaktant mit einer ersten Menge eines kristallinen Zeolith-Katalysators vom ZSM-5-Typ in einer Wirbelschichtbett-Reaktionszone unter exothermen Reaktionsbedingungen, um eine Alkoholumwandlung von 80—95% zu bewirken und 80—95% der gesamten exothermen Wärme freizusetzen, die während der im wesentlichen vollständigen Umwandlung des Alkohols freigesetzt wird,

(b) Abtrennen des Produktes aus der Wirbelschichtbett-Reaktionszone von der ersten Menge des Katalysators vom ZSM-5-Typ,

(c) Leiten eines ersten Anteils der ersten Menge des Katalysators vom ZSM-5-Typ durch eine Katalysator-Abkühlzone unter Abkühlbedingungen und von da aus zurück zu der Wirbelschichtbett-Reaktionszone,

(d) Leiten eines zweiten Anteils der ersten Menge des Katalysators vom ZSM-5-Typ durch eine Katalysator-Regenerierungszone und von da aus zurück zu der Wirbelschichtbett-Reaktionszone, dadurch gekennzeichnet, daß es die weiteren Stufen umfaßt:

(e) Kontaktieren des Produktes aus der Wirbelschichtbett-Reaktionszone mit einer zweiten Menge eines kristallinen Zeolith-Katalysators vom ZSM-5-Typ in einer Festbett-Reaktionszone unter exothermen Reaktionsbedingungen, die für die im wesentlichen völlige Alkoholumwandlung effektiv sind und Freisetzung von 5—20% der gesamten exothermen Wärme, die während der im wesentlichen völligen Umwandlung des niederen Alkohols freigesetzt wird, und

(f) Rückgewinnung eines Reaktanten-Produkts, das leichte $C_1$—$C_4$-Kohlenwasserstoffgase und $C_5$+ Kohlenwasserstoffe im Benzin-Siedebereich aus der Festbett-Reaktionszone.

2. Verfahren nach Anspruch 1, worin die Wirbelschichtbett-Reaktionszone aus Schritt (a) eine verdünnte Katalysatorphasen-Steigrohrreaktionszone ist.

3. Verfahren nach Anspruch 2, worin die Katalysatordichte in der verdünnten Katalysatorphasen-Steigrohrreaktionszone zwischen 16 und 240 kg/m³ (1 und 15 pounds per cubic foot) beträgt.

4. Verfahren nach Anspruch 1, worin die Wirbelschichtbett-Reaktionszone von Schritt (a) eine dichte Wirbelschichtbett-Katalysator-Reaktionszone ist.

5. Verfahren nach Anspruch 3, worin die Katalysatordichte in der dichten Wirbelschichtbettkatalysator-Reaktionszone zwischen 32 und 641 kg/m³ (2 und 40 pounds per cubic foot) beträgt.

6. Verfahren nach Anspruch 1, worin die Wirbelschichtbett-Reaktionszone von Schritt (a) eine verdünnte Katalysatorphasen-Steigrohrreaktionszone in Reihe mit einer dichten Wirbelschichtbettkatalysator-Reaktionszone ist.

7. Verfahren nach einem der vorstehenden Ansprüche, worin der Reaktant einen einwertigen $C_1$—$C_4$ Alkohol, einen zweiwertigen $C_2$—$C_4$ Alkohol oder eine Mischung davon mit einem verwandten mit Sauerstoff verbundenen Stoff ist.

8. Verfahren nach Anspruch 7, worin der einwertige Alkohol Methanol ist.

9. Verfahren nach einem der vorstehenden Ansprüche worin der Reaktant 0—50 Gew.-% Wasser enthält.

10. Verfahren nach einem der vorstehenden Ansprüche worin ein Teil der $C_1$—$C_4$ Kohlenwasserstoffgase von Schritt (f) in die Festbett-Reaktionszone bei einer Geschindigkeit eingegeben wird, die effektiv ist, um die Temperaturerhöhung entlang der Festbett-Reaktionszone auf nicht als mehr als 56°C (100°F) zu begrenzen.

**Revendications**

1. Procédé de conversion d'un réactif contenant de l'alcool en un hydrocarbure présentant un point d'ébullition supérieur à celui du réactif et comprenant des hydrocarbures dont les points d'ébullition sont situés dans l'intervalle des points d'ébullition des essences, qui consiste:

(a) à mettre le réactif au contact d'une première quantité de catalyseur à base de zéolite cristalline du type ZSM-5 dans une zone de réaction en lit fixe, dans des conditions favorisant une réaction exothermique, pour effectuer une conversion de 80 à 95% de l'alcool et libérer de 80 à 95% de la chaleur totale libérée dans la réaction exothermique se produisant pendant la conversion sensiblement complète de l'alcool;

(b) à séparer le produit de la zone de réaction en lit fluidisé de la première quantité de catalyseur du type ZSM-5;

(c) à faire passer une première portion de la première quantité de catalyseur de type ZSM-5 à travers une zone de refroidissement du catalyseur dans des conditions de refroidissement et à la ramener de là à la zone de réaction en lit fluidisé;

(d) à faire passer une deuxième portion de la première quantité de catalyseur de type ZSM-5 à travers une zone de régénération du catalyseur et à la ramener de là à la zone de réaction en lit fluidisé, procédé caractérisé en ce qu'il comprend en outre les étapes consistant à:

(e) mettre le produit provenant de la première zone de réaction en lit fluidisé au contact d'une seconde quantité de catalyseur à base de zéolite cristalline de type ZSM-5 dans une zone de réaction en lit fixe dans des conditions favorisant une réaction exothermique propre à effectuer une conversion sensiblement complète de l'alcool et à libérer de 5 à 20% de la chaleur totale libérée dans la réaction exothermique se produisant pendant la conversion sensiblement complète de cet alcool inférieur; et

(f) à récupérer un produit contenant des hydrocarbures gazeux légers en $C_1$ à $C_4$ et des hydrocarbures dont les points d'ébullition sont situés dans l'intervalle des points d'ébullition des essences en $C_5+$ provenant de la zone de réaction en lit fixe.

2. Procédé selon la revendication 1, dans lequel la zone de réaction en lit fixe de l'étape (a) est une zone de réaction dans laquelle se produit une ascension de la phase catalyseur diluée.

3. Procédé selon la revendication 2, dans lequel la densité du catalyseur dans la zone de réaction où se produit une ascension de la phase catalyseur diluée est comprise entre 16 et 240 kg/m³ (1 et 15 lb/pied cubique).

4. Procédé selon la revendication 1, dans lequel la zone de réaction en lit fluidisé de l'étape (a) est une zone de réaction en lit de catalyseur fluidisé dense.

5. Procédé selon la revendication 3, dans lequel la densité du catalyseur dans la zone de réaction en lit de catalyseur fluidisé est comprise entre 32 et 641 kg/m³ (2 to 40 lb/pied cubique).

6. Procédé selon la revendication 1, dans lequel la zone de réaction en lit fluidisé de l'étape (a) est une zone de réaction où a lieu une ascension de la phase catalyseur diluée disposée en série avec une zone de réaction en lit de catalyseur fluidisé dense.

7. Procédé selon l'une quelconque des revendications précédentes, dans laquelle le réactif contient un alcool monovalent en $C_1$ à $C_4$, un alcool divalent en $C_2$ à $C_4$ ou un mélange de ceux-ci avec un dérivé oxygéné proche.

8. Procédé selon la revendication 7, dans lequel l'alcool monovalent est le méthanol.

9. Procédé selon l'une quelconque des revendications précédentes, dans lequel le réactif contient de 0 à 50% en poids d'eau.

10. Procédé selon l'une quelconque des revendications précédentes, dans lequel on introduit une portion des hydrocarbures gazeux en $C_1$ à $C_4$ de l'étape (f) dans la zone de réaction en lit fixe à une vitesse propre à limiter l'élévation de température dans la zone de réaction en lit fixe à une valeur qui ne dépasse pas 56°C (100°F).

FIG.1

C$_4$ - GAS

C$_5$ + GASOLINE

WATER

FIG.2

FIG.3

TO FIXED BED REACTOR

TO FIXED BED REACTOR

0 099 650